(19) Europäisches Patentamt

European Patent Office

Office européen des brevets

(11) Publication number: **0 111 376 B1**

(12) # EUROPEAN PATENT SPECIFICATION

(45) Date of publication of patent specification: **13.03.91**   (51) Int. Cl.⁵: **C07C 29/15**, C07C 31/04, C01B 3/36

(21) Application number: **83201746.1**

(22) Date of filing: **12.12.83**

The file contains technical information submitted after the application was filed and not included in this specification

(54) Process for the preparation of methanol.

(30) Priority: **14.12.82 NL 8204820**

(43) Date of publication of application:
**20.06.84 Bulletin 84/25**

(45) Publication of the grant of the patent:
**13.03.91 Bulletin 91/11**

(84) Designated Contracting States:
**AT BE DE FR GB IT NL**

(56) References cited:
**US-A- 3 920 717**
**US-A- 3 962 300**

(73) Proprietor: **STAMICARBON B.V.**
**Mijnweg 1**
**NL-6167 AC Geleen(NL)**

(72) Inventor: **De Lathouder, Hans Christiaan**
**Seipgensstraat 42**
**NL-6164 HR Geleen(NL)**

EP 0 111 376 B1

## Description

The invention relates to a process for the preparation of methanol by converting a lower hydrocarbon or mixture of lower hydrocarbons to a first gas mixture consisting substantially of CO, $CO_2$ and $H_2$, by partial oxidation thereof with oxygen and optionally water, at a reactor outlet temperature of between 800 and 1500 °C and a pressure of between 40 and 150 bar, supplying said first gas mixture to a second reactor where it is converted to a second gas mixture containing methanol at a temperature of between 240 and 320 °C and a pressure of between 40 and 150 bar in the presence of a suitable catalyst, separating a third gas mixture consisting of the non converted gaseous components from said second gas mixture by cooling, controlling the molar ratio $H_2/(2CO + 3CO_2)$ in the combined said first gas mixture and the depleted third gas mixture, which combined mixture is to be supplied to said second reactor, expanding the crude methanol, obtained by said cooling of said second gas mixture to a lower pressure, and purifying said methanol, in which process the energy released during said partial oxidation and the conversion of said combined gas mixture to said methanol containing second gas mixture is at least in part used for the generation of steam.

Such a process is described in US-A-3920717. In this process synthesis gas is produced by partial oxidation of a hydrocarbonaceous feed in a non catalytic synthesis gas generator. The molar ratio $H_2/CO$ is subsequently adjusted by non catalytic thermal direct water-gas shift, whereafter the gas stream is cooled and purified preferably with a portion of the methanol product.

It is further known to convert lower hydrocarbons such as methane ethane and propane, for example natural gas, to a synthesis gas by passing the hydrocarbons with steam over a heated catalyst (for example based on nickel), at elevated temperature and increased pressure. In this process a gas mixture is formed which contains more hydrogen than the theoretical stoichiometric requirement for the preparation of methanol. A disadvantage of this process is the relatively high energy consumption. Moreover, after the preparation of methanol, unused hydrogen remains, which can in most cases be used only as a fuel gas for heating purposes.

The object of the invention is to provide a process for the preparation of methanol from lower hydrocarbons which has a low energy consumption and which does not require a higher investment than in the known processes.

The invention is characterized in that $CO_2$ is removed from the said third gasmixture in an amount that the molar ratio $H_2/(2CO + 3CO_2)$ in the said combined gas mixture is at least 0.90.

Preferably the amount of said steam being generated is substantially equivalent to the energy demand of an oxygen plant which supplies the oxygen needed for the said partial oxidation.

In summary, essential steps in the process are the partial oxidation of the hydrocarbon to a gas mixture containing CO, $CO_2$, $H_2$ and $H_2O$, the depletion of $CO_2$ from the gas mixture from the synthesis reactor, which is to be recycled, in an amount that the desired ratio of the components of the reactor feed is established, and the preparation of methanol therefrom in a reactor, whereby the heat of reaction, therfrom as well as the heat of reaction generated during the partial oxidation is converted to medium pressure or high-pressure steam.

Advantages of the process according to the invention are that the energy consumption per tonne of methanol produced is low, that the only by-product is carbon dioxide, that no steam reformer is required, that a separate water gas shift reactor is dispensed with, and that it. is possible to prepare a mixture of methanol and other alcohols which is pre-eminently suitable for application as engine fuel, mixed with for example petrol.

As process feedstock, methane, ethane or propane or a mixture thereof can be used. If required, the hydrocarbons can be desulphurised before being introduced into the process or at any stage in the process prior to the methanol synthesis. Advantageously the gas mixture has a pressure of between 40 and 150 bar, more in particular between 70 and 90 bar.

The partial oxidation can be effected by feeding the hydrocarbon with oxygen and water vapour to a reactor containing a suitable catalyst. This catalytic partial oxidation can be carried out at a reactor outlet temperature of between 800 and 1200 °C and a pressure of between 20 and 100 bar, usually 40-65 bar. The ratio of water molecules to carbon atoms can be between 1.0 and 2.5. In this case, the gas stream obtained has to be cooled in order to substantially remove the water present in it. More preferably, an uncatalysed partial oxidation is used. This can be carried out at a pressure of between 40 and 150 bar and, a reactor outlet temperature of between 1100 and 1500 °C. In such case the amount of water in the feed can be low. The gas mixture formed will then contain only a small amount of water, i.e. water supplied to the process and water formed during the oxidation, which need not be removed.

The fresh feed to the synthesis reactor or combination of reactors contains a relatively small amount of water. As a result of the shift reaction which occurs in the reactor besides the methanol synthesis, hydrogen and carbon dioxide are formed. The mixture of non-converted, gaseous components leaving the reactor is relatively rich in carbon dioxide. Thus it is easy to remove a portion of the carbon dioxide by washing. The gas mixture to be recycled might be recovered by cooling and expanding the reactor effluent, in which process crude methanol would be separated out as a liquid. Preferably, however, the effluent is cooled to, for example, between 10 and 40 °C and the gases are separated out at the pressure prevailing in the said second reactor. The gas mixture thus obtained can then be washed at roughly the synthesis-pressure. The washing step can be effected with any one of the known media for washing out carbon dioxide. Preferably, crude methanol is used as the washing medium, at a temperature of between -35 and +40 °C, preferably between 15 and 35 °C. The carbon-dioxide-containing methanol can be expanded to about atmospheric pressure, in which process carbon dioxide evolves. Of the separated-out gas from the synthesis reactor, a portion can be discharged as waste and a portion might be added to the hydrocarbon feed. Also, a portion of the gas might be recycled direct to the synthesis, without a washing step.

By washing it is achieved that the molar ratio $H_2/(2CO + 3 CO_2)$ in the reactor becomes at least 0.90 and preferably at least 0.95. When the ratio is lower, the synthesis becomes less efficient. For the preparation of pure methanol, a ratio of about 1, especially between 1.0 and 1.02, is desired. By choosing a slightly lower ratio, more higher alcohols are formed as by-products. A mixture of methanol with such higher alcohols is particularly suitable for application as engine fuel, mixed with petrol.

The methanol synthesis takes place in a reactor which is operated at a temperature of between 240 and 320 °C and a pressure of between 40 and 100 bar, in the presence of a suitable catalyst. Preferably, the pressure is between 70 and 90 bar and the temperature between 250 and 270 °C. The reactor design should enable the removal of the heat of reaction with generation of steam. Suitable reactors and reaction conditions are known and have been described in the literature. The known catalysts, e.g. catalysts based on oxides of copper, zinc, chromium and/or aluminium can be used.

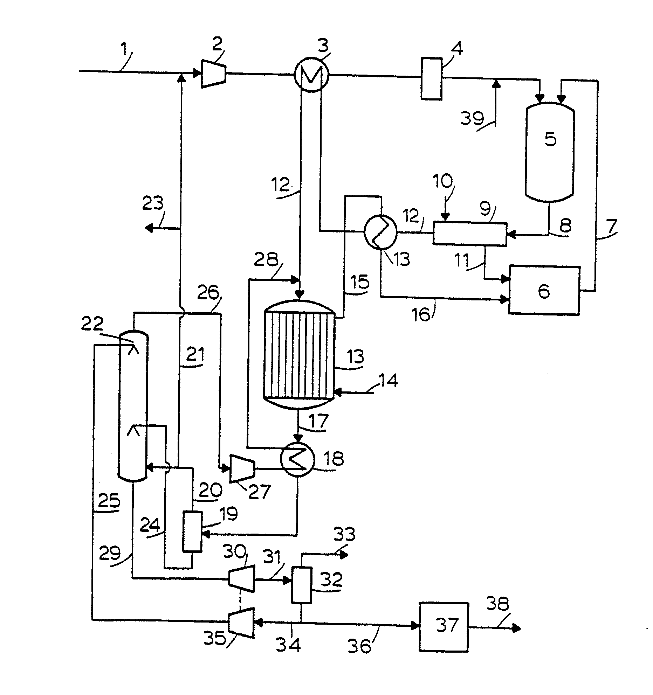

The process according to the invention will be described with reference to the figure. The gaseous hydrocarbon, preferably natural gas, is through line 1 fed to the partial oxidation reactor 5, via compressor 2, preheater 3 and desulphurisation unit 4. From an oxygen plant 6, oxygen is sent to the reactor through line 7. Through line 39, finally, a small amount of steam is supplied, for example such an amount that the $H_2O/C$ ratio is between 0.1 and 0.5.

The gas which leaves the reactor through line 8 is cooled in the steam generation unit 9, in which process the water supplied, through line 10 is converted to steam which is carried off through line 11. Through line 12, incorporating the heat exchanger 13, the gas is led to the synthesis reactor 13. The heat released in the conversion to methanol is used for the generation of steam. To this end, through line 14 water is supplied which is carried off as steam through line 15. In heat exchanger 13, this steam is further heated and then discharged through line 16. The reaction mixture leaves the reactor through line 17 and is cooled to a temperature of about 30 °C in heat exchanger 18 and, possibly, in further heat exchangers omitted from the drawing. In gas/liquid separator 19, the reaction mixture is separated into a gas substantially consisting of CO, $H_2$ and $CO_2$, with some methane and inert, which through line 20 is sent to the washer 22, and the crude methanol, which through line 24 is also sent to the washer. Through the branched line 21, a portion of the gas is recycled to the partial oxidation, and through line 23 a portion is discharged as waste, for example to a burner. The gas rid of a portion of the carbon dioxide is discharged from the washer through line 26, brought to synthesis pressure in compressor 27, if necessary, and recycled, through heat exchanger 18 and line 28. The crude methanol loaded with $CO_2$ is via turbine 30 expanded to about atmospheric pressure and is through line 31 led to the gas/liquid separator. The $CO_2$ is discharged from the separator through line 33. The crude methanol is discharged through line 34 and is in part supplied to the washer through pump 35 and line 25, another part being sent, through line 36, to the working-up section 37 from which the end product is discharged through line 38.

## Claims

1. Process for the preparation of methanol by converting a lower hydrocarbon or mixture of lower hydrocarbons to a first gas mixture consisting substantially of CO, $CO_2$ and $H_2$, by partial oxidation thereof with oxygen and optionally water, at a reactor outlet temperature of between 800 and 1500 °C and a pressure of between 40 and 150 bar, supplying said first gas mixture to a second reactor where it is converted to a second gas mixture containing methanol, at a temperature of between 240 and 320 °C and a pressure of between 40 and 150 bar in the presence of a suitable catalyst, separating a third gas mixture consisting of the non converted gaseous components from said second gas mixture by cooling, controlling the molar ratio $H_2/(2CO + 3CO_2)$ in the combined said first gas mixture and the depleted third gas mixture, which combined mixture is to be supplied to said second reactor, expanding the crude methanol, obtained by said cooling of said second gas mixture to a lower pressure, and purifing said methanol, in which process the energy released during said partial oxidation and the conversion of said combined gas mixture to said methanol containing second gas mixture is at least in part used for the generation of steam, characterized in that $CO_2$ is removed from the said third gasmixture in an amount that the molar ratio $H_2/(2CO + 3CO_2)$ in the said combined gas mixture is at least 0.90.

2. Process according to claim 1 wherein the amount of said steam being generated is substantially equivalent to the energy demand of an oxygen plant which supplies the oxygen needed for the said partial oxidation.

3. Process according to claim 1, wherein the said partial oxidation is carried out as a non-catalytic partial oxidation at a pressure of between 25 and 150 bar and a reactor outlet temperature of between 1100 and 1500 °C.

4. Process according to claim 1, characterized in that the said molar ratio $H_2/(2CO + 3CO_2)$ in the feed to the said second reactor is between 1.0 and 1.02.

5. Process according to claim 1, characterized in that the carbon dioxide is removed from said third gas mixture by washing with crude methanol.


**Revendications**

1. Procédé de préparation de méthanol, qui consiste à convertir un hydrocarbure inférieur ou un mélange d' hydrocarbures inférieurs en un premier mélange gazeux consistant sensiblement en CO, $CO_2$ et $H_2$, par oxydation partielle avec l'oxygène et, facultativement, avec de l'eau, à une température de sortie du réacteur comprise entre 800 et 1500 °C et sous une pression comprise entre 40 et 150 bars; à introduire le premier mélange gazeux dans un second réacteur dans lequel on le convertit en un second mélange gazeux contenant du méthanol à une température comprise entre 240 et 320 °C et sous une pression comprise entre 40 et 150 bars en présence d'un catalyseur convenable ; à séparer un troisième mélange gazeux consistant en composants gazeux non convertis provenant dudit second mélange gazeux par refroidissement ; à régler le rapport molaire $H_2/(2CO + 3CO_2)$ dans le premier mélange gazeux combiné avec le troisième mélange gazeux épuisé, ledit mélange gazeux devant être introduit dans ledit second réacteur ; à expanser le méthanol brut obtenu par ledit refroidissement dudit second mélange gazeux à une plus basse pression ; et à purifier ledit méthanol, procédé dans lequel l'énergie libérée pendant ladite oxydation partielle et la conversion dudit mélange gazeux combiné en second mélange gazeux contenant le méthanol est utilisée au moins en partie pour engendrer de la vapeur d'eau, caractérisé en ce qu'on soutire $CO_2$ dudit troisième mélange gazeux en une quantité telle que le rapport molaire $H_2/(2CO + 3CO_2)$ dans ledit mélange gazeux combiné soit d'au moins 0,90.

2. Procédé selon la revendication 1, dans lequel la quantité de ladite vapeur engendrée est sensiblement équivalente à la demande en énergie d'un atelier à oxygène qui fournit l'oxygène nécessaire pour ladite oxydation partielle.

3. Procédé selon la revendication 1, dans lequel on effectue ladite oxydation partielle sous forme d'une oxydation partielle non catalytique sous une pression comprise entre 25 et 150 bars et à une température de sortie du réacteur comprise entre 1100 et 1500 °C.

4. Procédé selon la revendication 1, caractérisé en ce que ledit rapport molaire $H_2/(2CO+3CO_2)$ dans l'alimentation dudit second réacteur est compris entre 1,0 et 1,02.

5. Procédé selon la revendication 1, caractérisé en ce qu'on soutire l'anhydride carbonique dudit troisième mélange gazeux en le lavant avec du méthanol brut.


**Ansprüche**

1. Verfahren zur Herstellung von Methanol durch Überführen eines niedrigeren Kohlenwasserstoffes oder einer Mischung von niedrigeren Kohlenwasserstoffen in eine erste Gasmischung, die im wesentlichen aus CO, $CO_2$ und $H_2$ besteht, durch partielle Oxidation hievon mit Sauerstoff und gegebenenfalls Wasser bei einer Reaktorauslaßtemperatur von 800 bis 1500 °C und einem Druck von 40 bis 150 bar, Einbringen dieser ersten Gasmischung in einen zweiten Reaktor, wo sie in eine methanolhaltige zweite Gasmischung übergeführt wird, bei einer Temperatur von 240 bis 320 °C und einem Druck von 40 bis 150 bar in Anwesenheit eines geeigneten Katalysators, Abtrennen einer dritten Gasmischung bestehend aus den nichtübergeführten gasförmigen Bestandteilen von der zweiten Gasmischung durch Abkühlen, Regulieren des Molverhältnisses $H_2/(2CO + 3CO_2)$ in der kombinierten ersten Gasmischung und der erschöpften dritten Gasmischung, welche kombinierte Mischung dann dem zweiten Reaktor zugeführt wird, Expandieren des rohen Methanols, erhalten durch Kühlen der zweiten Gasmischung bei einem niedrigeren Druck und Reinigen des Methanols, in welchem Verfahren die während der partiellen Oxidation und der Überführung der kombinierten Gasmischung in die methanolhaltige zweite Gasmischung freigesetzte Energie zumindest zum Teil für die Erzeugung von Dampf verwendet wird, dadurch gekennzeichnet, daß $CO_2$ von der dritten Gasmischung in einer solchen Menge entfernt wird, daß das Molverhältnis $H_2/(2CO + 3CO_2)$ in der kombinierten Gasmischung mindestens 0,90 beträgt.

2. Verfahren nach Anspruch 1, worin die Menge des erzeugten Dampfes im wesentlichen dem Energiebedarf einer Sauerstoffvorrichtung äquivalent ist, die den für die partielle Oxidation benötigten Sauerstoff zuführt.

3. Verfahren nach Anspruch 1, worin die partielle Oxidation als nicht-katalytische partielle Oxidation bei einem Druck von 25 bis 150 bar und einer Reaktorauslaßtemperatur von 1100 bis 1500 °C durchgeführt wird.

4. Verfahren nach Anspruch 1, dadurch gekennzeichnet, daß das Molverhältnis $H_2/(2CO + 3CO_2)$ in der Zufuhr zum zweiten Reaktor 1,0 bis 1,02 beträgt.

5. Verfahren nach Anspruch 1, dadurch gekennzeichnet, daß das Kohlendioxid von der dritten Gasmischung durch Waschen mit rohem Methanol entfernt wird.

EP 0 111 376 B1

6